**Europäisches Patentamt**

**European Patent Office**

**Office européen des brevets**

(11) Publication number : **0 486 294 A2**

# (12) EUROPEAN PATENT APPLICATION

(21) Application number : **91310501.1**

(22) Date of filing : **14.11.91**

(51) Int. Cl.⁵ : **C08L 5/08,** C08L 29/04, C08L 39/06, A61L 33/00

(30) Priority : **15.11.90 US 613599**

(43) Date of publication of application :
**20.05.92 Bulletin 92/21**

(84) Designated Contracting States :
**CH DE ES FR GB IT LI LU NL**

(71) Applicant : **IOLAB CORPORATION**
**500 Iolab Drive**
**Claremont, CA 90711 (US)**

(72) Inventor : **Shah, Kishore R.**
**568 Cabot Hill Road**
**Bridgewater, NJ 08807 (US)**
Inventor : **Mowbray, Samuel L.**
**3913 Northampton Avenue**
**Claremont, CA 91711 (US)**

(74) Representative : **Fisher, Adrian John et al**
**CARPMAELS & RANSFORD 43 Bloomsbury**
**Square**
**London WC1A 2RA (GB)**

(54) **Compatible blends containing chitosan.**

(57) Miscible blends of chitosan having a degree of N-acetylation between about 30 to about 60 percent, and a water soluble polymer.
Blends are useful for preparing medical devices, especially ophthalmic devices such as a corneal bandage contact lens.

EP 0 486 294 A2

This invention relates to compositions containing derivatives of chitosan, which is itself a deacetylated form of the naturally occurring aminopolysaccharide chitin. More particularly, it relates to compositions containing reacetylated chitosan for use in the preparation of numerous medical devices, such as corneal bandage contact lenses.

Chitin and its derivatives, including chitosan, have long been known to have numerous potential beneficial uses. For example, U.S. Patent 3,632,754 discloses the use of chitin for accelerating and promoting wound healing. U.S. Patent 3,988,411 discloses the use of poly(N-acetyl-D-glucosamine), which is the major component of chitin, as a material of construction for surgical sutures. These sutures are said to be bioabsorbable because of enzymatic degradation which occurs in bodily tissue when the suture contacts the enzyme lysozyme. Other examples of potentially interesting applications are disclosed in Miyazaki et al., Chem. Pharm. Bull. 29(10)3067-3069(1981), which describes the use of chitin and chitosan as drug carriers, and European patent Application 0 356 060, which describes blends containing chitosan for use as ulcer dressings.

Of particular interest are the uses of derivatives of chitin and chitosan described in U.S. Patent Nos. 4,532,267 (Allan patent) and 4,447,562 (Ivani patent). The Allan patent discloses the preparation of a vision correcting contact lens from a derivative of chitin or chitosan, or the ester, ether or amide of such a derivative. The Ivani patent discloses the preparation of absorbable graft or block copolymer blends of a chitin derivative and a selected monomer or prepolymer. These absorbable polymer blends are said to be useful for the preparation of numerous medical devices and pharmaceutical compositions. The pharmaceutical compositions include ophthalmic compositions and preparations, and most notably include the preparation of contact lenses.

Although the various derivatives of chitin and chitosan, as well as blends of such derivatives with additional components, have potential uses for numerous medical applications, certain problems with the use of these compositions still exist. Absorbable medical devices which come into contact with, or are lodged in, various parts of the human anatomy need to exhibit a controlled rate of bioabsorption. Chitin is bioabsorbable but its bioabsorption characteristics are defined by its structure and are not controllable. In addition, it has very poor solubility in most suitable solvents such as water and dilute acids. Consequently, processing of chitin into desirable forms is rendered difficult. On the other hand, chitosan, which has free amino groups, exhibits the solubility characteristics required for ease of processing.

Unfortunately, the rate of bioabsorption of chitosan, which is linked to the enzyme lysozyme, depends in part on the degree of N-acetylation of the chitosan derivative. The degree of N-acetylation of commercially available chitosan is unsatisfactory for numerous applications, especially ophthalmic applications. Therefore, a need exists in the medical arts to develop a modified derivative of chitosan, in conjunction with other compatible components, which will exhibit a controlled rate of bioabsorption to meet the specific requirements of its intended use.

The invention is a composition comprising a mixture of a chitosan having a degree of N-acetylation between about 30 to about 60 percent, and a water-soluble polymer.

The partially reacetylated chitosan is miscible with the water-soluble polymer over a wide concentration range and yields an optically clear solution in dilute aqueous acid. The addition of water-soluble polymer to the chitosan not only creates a compatible blend but also enhances the biodegradation rate and hydration of the blend in comparison to that of the modified chitosan alone.

Compatible blends of chitosan and water-soluble polymer which form the composition of this invention can be cast from solution and subsequently machined or fabricated to prepare numerous medical and ophthalmic devices. Surprisingly, the rate of enzymatic biodegradation is faster for such devices relative to that of a device derived from a blend of unmodified chitosan and water-soluble polymer.

The compositions of this invention can be used in those applications where chitin or chitosan and its derivatives are currently being explored or used. For example, the compositions can be used for numerous medical and ophthalmic applications such as coatings for surgical sutures, implantable drug delivery matrices, coatings for vascular grafts, wound dressings, and most importantly, for the preparation of corneal bandage contact lenses.

For purposes of describing this invention, chitosan is the acid-soluble deacetylated derivative of chitin, which is a naturally occurring amino polysaccharide found in crustacean shells. See, for example, U.S. patent 3,770,673. The degree of N-acetylation for chitin is typically greater than 90 percent, and in most cases chitin is fully acetylated. The preferred chitosan used for preparing the blends of this invention is an ulta-pure grade of chitosan sold as Pronova™ chitosan, which generally has a degree of N-acetylation between about 15 to about 25 percent. The degree of N-acetylation can be measured using infrared spectroscopy as described in Domszy et al., Makromol. Chem., Vol. 186, No. 8, pp. 1671-1677 (1985).

The reacetylation of chitosan to achieve a degree of N-acetylation between about 30 to about 60 percent can be carried out using known techniques as described, for example, in Hirano et al., Biomaterials, Vol. 10, pp. 574 (1989). Chitosan having the same degree of acetylation can also be obtained by controlled hydrolysis of chitin. Generally, a degree of N-acetylation less than about 30 percent undesirably decreases the biodegradation rate of articles or devices prepared from the blends of this invention, especially ophthalmic devices such as a corneal bandage contact lens. A degree of N-acetylation greater than about 60 percent significantly reduces the solubility of the chitosan in conventional solvents, particularly dilute organic acids, and therefore the processing of the chitosan with the water-soluble polymer will often result in undesirable gel formation. The preferred degree of N-acetylation for the chitosan, especially for a corneal bandage contact lens, is between about 30 to about 50 percent.

A "water-soluble" polymer, as the term is used in this description, is a polymer which readily forms a homogeneous solution in water. For best processing characteristics, the water-soluble polymer component of the blend preferably has a weight average molecular weight greater than about 40,000 as measured by gel permeation chromatography. The preferred water-soluble polymers are poly(vinyl pyrrolidone) (PVP), polyacrylamide, poly(N,N-dimethylacrylamide), poly(vinyl alcohol), and cellulosic derivatives such as hydroryethyl cellulose and hydroxypropyl methyl cellulose. The most preferred water-soluble polymer is a pharmaceutical grade of poly(vinyl pyrrolidone).

The ratio of partially acetylated chitosan to water-soluble polymer in the blend can vary over a wide range because the chitosan is compatible with the water-soluble polymer at low and high concentrations. Generally, the desired ratio of partially acetylated chitosan to water-soluble polymer will depend on the particular application, and can be readily determined empirically. The weight ratio can vary from about 95:5 to about 5:95. The preferred weight ratio, particularly for the preparation of a corneal bandage lens, is between about 70:30 to about 30:70.

In one embodiment, a plasticizer or excipient such as a polyalkylene oxide, e.g. polyethylene glycol (PEG), can be incorporated into the compatible blend of the chitosan and water-soluble polymer. Such an additive can aid processing, impart desired characteristics to a finished medical device, or control the rate of release of various substituents dispersed or solubilized in the blend.

Although the blends of this invention can be processed to prepare numerous medical devices using conventional techniques, the blends are particularly well adapted for the preparation of contact lenses, particularly corneal bandage lenses. In one embodiment, these lenses can be prepared by first casting a solution of the blend in a dilute organic acid onto a suitable surface and then machined. These conventional processing techniques are described in detail in the Allan patent.

Once a lens is prepared, it is typically neutralized with dilute aqueous base, and then washed with water or saline. In this manner, the softness, nonirritability and oxygen permeability of the lens are improved. The actual oxygen permeability and water content of the lens can be adjusted by adjusting the composition of the blend. The desired bioabsorption rate, which is preferably less than 7 days for complete degradation, can be obtained by appropriately tailoring the degree of N-acetylation of the reacetylated chitosan.

The following examples illustrate the practice of the invention, but are not intended to limit the scope of the claimed invention. Additional embodiments within the scope of the claimed invention will become readily apparent to those skilled in the art.

EXAMPLES

Preparation of Chitosan Base

A 5.0 gallon stainless steel vessel equipped with a mechanical stirrer (plastic agitator) is charged with 12.50 liters of concentrated ammonium hydroxide (28-30% $NH_3$).

To the above solution is added 300.00 g of chitosan glutamate (Batch 809-572-01 from Protan Inc.). The reactants are allowed to stir for 4 hours after which time the entire contents are filtered through a course sintered glass funnel. After filtration, the solids are washed with 1 x 10 liters of distilled water to remove the excess ammonium hydroxide and the glutamate salts. Finally, the solids are washed with 5 x 500 ml of acetone. After air drying, the solids are transferred to a glass container and freeze dried for a period of 72 hours. The yield is 154.00 g. The inherent viscosity of the chitosan base for each of two runs is 7.74 and 7.46, respectively, as measured in an aqueous mixture of 0.20 molar sodium chloride and 0.1 molar acetic acid.

Acetylation of Chitosan Base (50 mole % Acetylated Chitosan)

Prior to acetylation, films of the chitosan are cast from 2 wt. % acetic acid (e.g. 0.10 g of chitosan per 20.0 g of 2 wt. % acetic acid) and air dried. The amount of residual (molar) acetyl groups is then determined by the infrared spectroscopy. This allows for the exact amount of acetic anhydride to be used for the acetylation. By the analytical IR method the chitosan base is shown to contain 19 mole % acetyl groups.

To a 5 gallon plastic pail equipped with a stainless steel agitator is added 800 mls of 2 wt. % acetic acid. To this solution is added (slowly) 40.0 gms of chitosan base which contains 19 mole % acetyl groups. The mixture is stirred overnight and the next day 4000 mls of high-purity methanol is added. After stirring an additional 2 hours, 7.86 g of acetic anhydride (99%) (0.77 moles) is added dropwise over 1/2 hour. The calculated total amount of acetylation is 50 mole %. The reaction mixture is allowed to set overnight. The next day, the solution is poured into a large plastic tray and allowed to evaporate in the hood. Finally, the solids are placed in vacuum at 40°C for 24 hours. The yield is 43.0 g. Films are cast in 2 wt. % acetic acid for the IR analyses. Infrared analysis of the film samples indicates a degree of N-acetylation of 50%.

Preparation of Films from Comptabible Blends of Chitosan Base and PVP

Film Preparation:

For each of the three compositions shown in Table 1, appropriate amounts of PVP, chitosan having a degree of N-acetylation of 50%, and PEG 400 were dissolved in 4% aqueous acetic acid by stirring for 48+ hours at room temperature to obtain a 5-10% solids solution. The resultant solutions were poured into separate polyethylene trays and air dried to obtain PEG plasticized films of the PVP/chitosan blends. These films were then placed in 1 N sodium hydroxide solution for 4-5 hours, and then extensively washed with normal saline until the washings were neutral. The fully hydrated polymer blend films thus obtained had thicknesses in the range of 0.5 to 1.0 mm. These films were stored in normal saline for further testing.

Enzymatic Degradation Test

Hen egg lysozyme (HEL), dissolved in phosphate buffer solution at a pH of 7.2, was used as a medium at 37°C to study enzymatic degration of the PVP/Chitosan blend films. One centimeter diameter sections of films of each composition were tested for degradation at the enzyme concentrations of 0, 1, 2, and 3 mg/ml. The test specimens were incubated in the enzymatic media at 37°C for a period of 30 hours, and observations were made at different time intervals during this period. The results are shown in Table 1.

EP 0 486 294 A2

## TABLE 1

### ENZYMATIC DEGRADATION OF BLENDS OF PVP AND
### 50% ACETYLATED CHITOSAN

### Incubation Time at 37°C

| PVP/ CHITOSAN RATIO | HEL CONC mg/ml | 5 | 6 | 7 | HOURS 8 | 24 | 26 | 28 | 30 |
|---|---|---|---|---|---|---|---|---|---|
| 90:10 | 0 | I | I | I | I | I | I | I | I |
|  | 1 | I | I | I | F/S | D |  |  |  |
|  | 2 | I | D |  |  |  |  |  |  |
|  | 3 | I | D |  |  |  |  |  |  |
| 80:20 | 0 | I | I | I | I | I | I | I | I |
|  | 1 | I | I | I | I | D |  |  |  |
|  | 2 | I | I | I | I | D |  |  |  |
|  | 3 | I | I | I | I | D |  |  |  |
| 70:30 | 0 | I | I | I | I | I | I | I | I |
|  | 1 | I | I | I | I | F/L | F/L | F/M | D |
|  | 2 | I | I | I | I | F/M | F/M | F/S | D |
|  | 3 | I | I | I | I | F/S | F/S | D |  |

| | |
|---|---|
| HEL | Hen egg lysozyme |
| I | Intact |
| F/S | Fragmented (Small Pieces) |
| F/M | Fragmented (Medium Pieces) |
| F/L | Fragmented (Large Pieces) |
| D | Dissolved |

The results from Table 1 indicate that:

(a) No degradation of the blend occurred in the absence of the enzyme. All the compositions dissolved in the medium in the presence of enzyme within 30 hours.

(b) The greater the enzyme concentration, the more rapid was the sample dissolution.

(c) The greater the proportion of PVP in the blend, the more rapidly the sample dissolved.

Surprisingly, the same blend ratios of chitosan/PVP using a commercially available chitosan having a degree of N-acetylation of between 15-20% remains essentially intact under the above experimental conditions after 30 hours.

Percent Hydration Test

Fully hydrated film samples for chitosan/PVP films (70/30 chitosan/PVP weight ratio) are prepared in a manner similar to that described above for the enzymatic degradation study of 50% acetylated chitosan (Table 1). The percent water at equilibrium of each of the film samples prepared is shown in Table 2.

5

TABLE 2

HYDRATION AND LINEAR EXPANSION OF 70/30 CHITOSAN/PVP
FILMS AS A FUNCTION OF PERCENTAGE ACETYLATION

| PERCENT ACETYLATION | LINEAR EXPANSION | % $H_2O$ (Equilibrium Water Content) |
|---|---|---|
| 31 | 1.15 | 75.6 |
| 37 | 1.27 | 84.5 |
| 50 | 1.38 | 86.9 |

The results clearly illustrate an increase in water uptake at equilibrium as the degree of N-acetylation of the chitosan increases. This is a desirable effect for numerous medical applications.

**Claims**

1. A composition comprising a mixture of a chitosan having a degree of N-acetylation between about 30 to about 60 percent, and a water-soluble polymer.

2. The composition of claim 1 wherein the chitosan has a degree of N-acetylation between about 30 to about 50 percent.

3. The composition of claim 1 or claim 2 wherein the water-soluble polymer has a weight average molecular weight greater than about 40,000.

4. The composition of any preceding claim wherein the water-soluble polymer is poly(vinyl alcohol).

5. The composition of any one of claims 1 to 3 wherein the water-soluble polymer is a pharmaceutical grade of poly(vinyl pyrrolidone).

6. The composition of any preceding claim wherein the weight ratio of reacetylated chitosan to water-soluble polymer is between 95:5 to about 5:95.

7. The composition of any preceding claim wherein the weight ratio of reacetylated chitosan to water-soluble polymer is between about 70:30 to about 30:70.

8. A corneal bandage lens prepared from a composition according to any preceding claims.

9. The corneal bandage lens of claim 8 wherein substantially completely biodegradation of the lens occurs in less than 7 days.